# EUROPEAN PATENT SPECIFICATION

(11) **EP 2 281 195 B1**
(45) Date of publication and mention of the grant of the patent: **22.02.2012**
(21) Application number: 09738132.1
(22) Date of filing: 28.04.2009
(51) Int. Cl.: G01N 33/53, G01N 33/574

(54) **PRO-EPIL EXPRESSION LEVEL IN A BIOLOGICAL SAMPLE AS TESTICULAR CANCER BIOMARKER, PARTICULARLY IN COMBINATION WITH THE HCGBETA AND AFP BIOMARKERS**
PRO-EPIL-EXPRESSIONSNIVEAU IN EINER BIOLOGISCHEN PROBE ALS BIOMARKER FÜR HODENKREBS, INSBESONDERE IN KOMBINATION MIT DEN BIOMARKERN HCGBETA UND AFP
NIVEAU D EXPRESSION DE PRO-EPIL DANS UN ÉCHANTILLON BIOLOGIQUE EN TANT QUE BIOMARQUEUR DU CANCER DES TESTICULES, EN PARTICULIER EN COMBINAISON AVEC LES BIOMARQUEURS HCGBETA ET AFP

(30) Priority: 28.04.2008 US 48412 P
(43) Date of publication of application: 09.02.2011
(73) Proprietor: Université Paris Descartes, 75006 Paris (FR); Institut Curie, 75005 Paris (FR); Institut Gustave Roussy, 94800 Villejuif (FR); Universita Degli Studi di Siena, 53100 Siena (IT); Centre National de la Recherche Scientifique CNRS, 75016 Paris (FR)
(72) Inventor: BELLET, Dominique, F-92100 Boulogne (FR); PECKING, Alain, F-92700 Colombes (FR); RICHON, Sophie, F-95290 L'isle Adam (FR); PETRAGLIA, Felice, I-53100 Siena (IT)
(74) Representative: Faivre Petit, Frédérique
(86) International application number: PCT/EP2009/055100
(87) International publication number: WO 2009/133088

(56) References cited:
- WO-A-99/09172
- US-A- 5 910 480
- FOTTNER CHRISTIAN ET AL: "Elevated serum levels of IGF-binding protein 2 in patients with non-seminomatous germ cell cancer: correlation with tumor markers alpha-fetoprotein and human chorionic gonadotropin" EUROPEAN JOURNAL OF ENDOCRINOLOGY, vol. 159, no. 3, September 2008 (2008-09), pages 317-327, XP009119475 ISSN: 0804-4643
- NEUVIANS TANJA FLASCALE ET AL: "Differential expression of IGF components and insulin receptor isoforms in human seminoma versus normal testicular tissue" NEOPLASIA (NEW YORK), vol. 7, no. 5, May 2005 (2005-05), pages 446-456, XP002535944 ISSN: 1522-8002
- AIGNER A ET AL: "Marked increase of the growth factors pleiotrophin and fibroblast growth factor-2 in serum of testicular cancer patients." ANNALS OF ONCOLOGY : OFFICIAL JOURNAL OF THE EUROPEAN SOCIETY FOR MEDICAL ONCOLOGY / ESMO OCT 2003, vol. 14, no. 10, October 2003 (2003-10), pages 1525-1529, XP002535945 ISSN: 0923-7534
- BRANDT B ET AL: "Expression of early placenta insulin-like growth factor in breast cancer cells provides an autocrine loop that predominantly enhances invasiveness and motility" ENDOCRINE-RELATED CANCER, vol. 12, no. 4, December 2005 (2005-12), pages 823-837, XP002535946 ISSN: 1351-0088

## Description

The present invention is directed to the use of the expression level of the pro-EPIL gene as a biomarker for the diagnosing of testicular cancer, particularly a testicular germ cell tumor. The invention also relates to an in vitro method for detecting and/or classifying a testicular cancer in a subject comprising a step of determining the expression level of the gene encoding the pro-EPIL peptide in a biological, particularly in combination with the determination of the beta subunit HCGβ and the human alpha-fetoprotein AFP. The invention is also directed to a kit or solid support comprising nucleic acids or antibodies capable of determining the presence or the expression level of these three biological markers.

Management of patients with testicular cancers is a success in modem oncology, since more than 90 percent of patients with newly diagnosed germ-cell tumors are cured and testicular cancer mortality rates have fallen by about 70 % in the USA and western Europe since the 1970s.¹⁻³ This success is due to two major improvements in management of these neoplasms: the introduction of cisplatin-containing combination chemotherapy that dramatically improved the cure rate, and simultaneous introduction of serum tumor markers, namely human chorionic gonadotropin (HCG), its free beta subunit (HCGβ) and alpha-fetoprotein (AFP). These tumor markers help in diagnosis and play an important role in assessment of response to treatment and in monitoring remission.⁴ Lactate dehydrogenase (LDH) is also a germ cell tumor product, but it is a less specific tumor marker.

One factor that affects prognosis is the delay in diagnosing testicular tumors, which impacts the stage and therefore the prognosis. During the last two decades, there has been a trend over time towards reduction of diagnostic delay.⁵ However, recent results of a large population-based study show that long diagnostic delay is associated with lower survival for patients with non-seminomatous tumors.⁶ Moreover, the worldwide incidence of testicular cancer has more than doubled over the past 40 years. Recently, its incidence has been rising in nearly all industrialized countries.⁷ Despite improvements that occurred 30 years ago, the decline in mortality rates has begun to slow in the past few years in Europe, the USA and Japan, indicating the possible approach of an asymptote in testis cancer mortality.³ Patients with advanced disease must be treated with chemotherapy regimens that have substantial toxicity, and some of these patients fail to respond to such treatment.

In this context, it would be worthwhile to further reduce diagnostic delay and to continue to improve treatment modalities for testicular tumors so as to reduce the extent of such therapy and the burden of their toxicity. Novel biomarkers might contribute to achieving such a goal.

This is the object of the present invention.

The inventors have demonstrated that pro-EPIL peptide, or specific fragments thereof, can be used as a tumor biomarker for detecting and/or classifying a testicular cancer in a subject, particularly as a class of testicular germ cell tumor.

The EPIL peptide is encoded by the insulin-like 4 gene (INSL4). This gene was identified by screening a cDNA library of first-trimester human placenta. INSL4 is highly expressed in early placenta and, with the exception of faint expression in normal uterine tissues, expression of INSL4 transcripts was not detected in any other normal tissue tested thus far.⁸ The early placenta insulin-like peptide (EPIL) encoded by the insulin-like 4 gene is a member of the insulin-related gene family comprising insulin, relaxin (RLX), insulin-like growth factors 1 and II (IGFI I and IGF II), Leydig insulin-like peptide (LEY I-L) encoded by the INSL3 gene and peptides encoded by INSL5 and INSL6 genes.

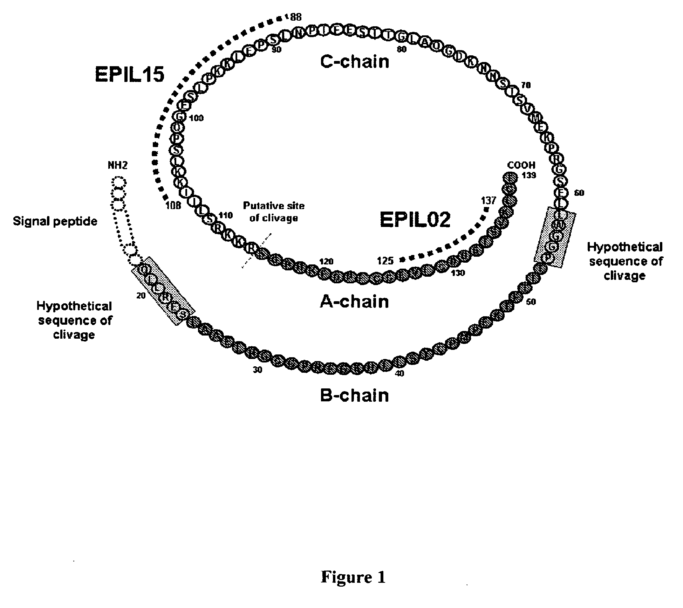

EPIL is a 139-amino-acid polypeptide which is synthesized as a preprohormone characterized by a signal peptide, a B-chain, a connecting C-peptide and a terminal A-chain (Figure 1). In placenta, it was found that trophoblast cells translate INSL4 mRNAs into immunoreactive pro-EPIL peptides comprising the B-, C- and A-chains.⁹ Initially, pro-EPIL peptide was detected in amniotic fluid and maternal serum during normal pregnancy, and the excretion pattern of pro-EPIL was similar to that of HCGβ, suggesting common regulation pathways.¹⁰

The inventors have demonstrated that pro-EPIL peptide (PEP) is expressed and secreted by testicular germ cell tumors (TGCTs). PEP expression was investigated in patients with testicular cancers at the cellular and serum levels, enabling to show that PEP is a novel biomarker which provides additional information in comparison to that provided by HCGβ and AFP.

Thus, the present invention is directed to an in vitro method for detecting and/or classifying a testicular cancer in a subject, comprising the step of determining the expression level of the gene encoding the pro-EPIL peptide in a biological sample isolated from said subject wherein overexpression of said gene encoding the pro-EPIL peptide is indicative of the presence of a testicular cancer and/or class of testicular germ cell tumor.

In a preferred embodiment, the method according to the invention further comprises a step of determining the expression level of at least one gene selected from the group of gene consisting of the genes encoding the human gonadotropin HCG, its beta subunit HCGβ and the human alpha-fetoprotein AFP in a biological sample isolated from said subject wherein overexpression of at least one of said gene encoding the encoding the HCG, its beta subunit HCGβ or the human AFP is indicative of the presence of a testicular cancer and/or class of testicular germ cell tumor.

In a more preferred embodiment, said testicular cancer and/or class of testicular germ cell tumor is seminatous or non-seminatous.

The methods for determining the expression level of the genes encoding the human pro-EPIL, the human HCG, or its subunit HCGβ, and the human AFP in a biological sample isolated from said subject are well known by the skill person.

In a more preferred embodiment, in the method according to the invention, the presence of an overexpression of the gene encoding the pro-EPIL peptide, an overexpression of the gene encoding the HCGβ and an overexpression of the gene encoding the alpha-fetoprotein AFP in a biological sample isolated from said is indicative of the presence of a testicular cancer and/or class of testicular germ cell tumor of non-seminatous form.

In another aspect, the present invention is directed to a method for identifying a compound candidate for a pharmacological agent useful in the treatment of testicular cancer, particularly a testicular germ cell tumor, comprising the step of:
a) contacting a non-human mammal subject presenting a testicular cancer, particularly a testicular germ cell tumor, with a candidate pharmacological agent;
b) determining the expression level of the gene encoding the pro-EPIL peptide in a biological sample isolated from said subject;
c) optionally, further determining the expression level of at least one gene selected from the group of gene consisting of the genes encoding the HCG, its subunit HCGβ and the human AFP in a biological sample isolated from said subject,
wherein a decrease in the test amount of expression of the gene encoding the pro-EPIL peptide, and optionally, a decrease of the expression of the gene determined in step c), indicates that the candidate pharmacological agent is a potential compound for a pharmacological agent useful in the treatment of testicular cancer, particularly a testicular germ cell tumor.

In another aspect, the present invention is directed to a method for evaluating the effect in a subject of a treatment for testicular cancer, particularly a testicular germ cell tumor, comprising the step of:
a) determining the expression level of the gene encoding the pro-EPIL peptide in a biological sample isolated from said subject;
b) determining a second expression level of the gene encoding the pro-EPIL peptide in a biological sample isolated from said subject;
c) comparing the first and second amounts of expression level of the gene encoding the pro-EPIL peptide in a biological sample isolated from said subject;
wherein a decrease of the expression level of the gene encoding the pro-EPIL peptide in the second biological sample isolated from said subject indicates the regression of said testicular cancer, particularly a testicular germ cell tumor.

In the methods according to the present invention as defined above, it is preferred that the expression level is determined by detecting the presence, absence or level of mRNA transcribed from said INSL4 gene or of pro-EPIL peptide encoded by said gene, or specific peptidic fragment thereof. The detection of the presence, absence or level of pro-EPIL peptide encoded by said gene, or specific peptide fragment thereof is more preferred.

The amino-acid and mRNA sequence of the human Early placental insulin-like protein (INSL4 or EPIL, see GenPep accession number NP_002186) is:
Complete pro-EPIL amino acids sequence (SEQ ID NO: 1)

| | |
|---|---|
| aa1-22: | signal peptide (SEQ ID NO: 2) |
| aa23-52: | "B chain" (SEQ ID NO: 3) |
| aa59-108: | "C chain (SEQ ID NO: 4) |
| aa115-139: | "A chain" (SEQ ID NO: 5). |

Insulin-like 4 (placenta) (INSL4), mRNA (see Genbank accession number NM_002195, SEQ ID NO: 6
nt106-522: pro-EPIL coding sequence (SEQ ID NO: 7)
nt106-171: signal peptide (SEQ ID NO: 8)
nt172-261: "B chain" (SEQ ID NO: 9)
nt280-429: "C chain (SEQ ID NO: 10)
nt448-522: "A chain » (SEQ ID NO: 11).

INSL4 gene encodes a precursor that undergoes post-translational cleavage to produce 3 polypeptide chains, A-C, that form tertiary structures composed of either all three chains, or just the A and B chains (Chassin,D., Laurent, A., Janneau,J.L., Berger,R. and Bellet, D., Genomics 29 (2), 465-470 (1995)).

It shall be understood that the term "specific peptide fragment" designates in particular a fragment of an amino acid sequence of a polypeptide having at least one of the functional characteristics or properties of the complete polypeptide, notably in that it is capable of being recognized by a specific antibody and/or that the expression level of such a specific peptide fragment is correlated to expression level of the complete or partial pro-EPIL expressed.

It is understood that the term "specific peptide fragment" designates particularly a polypeptide including a minimum of 9 amino acids, preferably 10, 11 or 12 amino acids, and most preferably 15, 20 or 25 amino acids of the sequence SEQ ID No: 1, preferably this fragment contains a fragment of at least the chain A, B or C of the human pro-EPIL.

Specific anti-pro-EPIL monoclonal or polyclonal antibodies are available to the skilled man. An isolated pro-EPIL, or a specific fragment thereof, can be used as an immunogen to generate antibodies that bind such protein using standard techniques for polyclonal and monoclonal antibody preparation. It may be also possible to use any fragment of these protein which contains at least one antigenic determinant may be used to generate these specific antibodies.

A protein immunogen typically is used to prepare antibodies by immunizing a suitable subject, (e.g., rabbit, goat, mouse or other mammal) with the immunogen. An appropriate immunogenic preparation can contain said pro-EPIL polypeptide, or fragment thereof, and further can include an adjuvant, such as Freund's complete or incomplete adjuvant, or similar immuno-stimulatory agent.

Thus, antibody for use in accordance with the invention include either polyclonal, monoclonal chimeric or humanized antibodies. antibodies able to selectively bind, or which selectively bind to an epitope-containing a pro-EPIL polypeptide comprising a contiguous span of at least 9 to 10 amino acids of a pro-EPIL fragment, particularly of a fragment of at least the chain A, B or C of the human pro-EPIL.

A preferred agent for detecting and quantifying mRNA or cDNA encoding the human pro-EPIL is a labeled nucleic acid probe or primers able to hybridize this mRNA or cDNA. The nucleic acid probe can be an oligonucleotide of at least 10, 15, 30, 50 or 100 nucleotides in length and sufficient to specifically hybridize under stringent conditions to the mRNA or cDNA. The nucleic acid primer can be an oligonucleotide of at least 10, 15 or 20 nucleotides in length and sufficient to specifically hybridize under stringent conditions to the mRNA or cDNA, or complementary sequence thereof.

A preferred agent for detecting and quantifying the human pro-EPIL, is an antibody able to bind specifically to this protein, preferably an antibody with a detectable label. Antibodies can be polyclonal, or more preferably, monoclonal. As used herein, the term encompasses not only intact polyclonal or monoclonal antibodies, but also fragments thereof (such as Fab, Fab', F(ab')2, Fv), single chain (ScFv), mutants thereof, fusion proteins comprising an antibody portion, humanized antibodies, chimeric antibodies, diabodies linear antibodies, single chain antibodies, multispecific antibodies (e.g., bispecific antibodies) and any other modified configuration of the immunoglobulin molecule that comprises an antigen recognition site of the required specificity. An antibody includes an antibody of any class, such as IgG, IgA, or IgM (or sub-class thereof), and the antibody need not be of any particular class.

The term "labeled", with regard to the probe or antibody, is intended to encompass direct labeling of the probe or antibody by coupling (i.e., physically linking) a detectable substance to the probe or antibody, as well as indirect labeling of the probe or antibody by reactivity with another reagent that is directly labeled. Examples of indirect labeling include detection of a primary antibody using a fluorescently labeled secondary antibody and end-labeling of a DNA probe with biotin such that it can be detected with fluorescently labeled streptavidin.

For example, in vitro techniques for detection of mRNA include Northern hybridizations and in situ hybridizations. In vitro techniques for detection of the candidate protein include enzyme linked immunosorbent assays (ELISAs), Western blots, immunoprecipitations and immunofluorescence. In vitro techniques for detection of candidate cDNA include Southern hybridizations.

When the invention encompasses kits for quantifying the level of the human pro-EPIL polypeptide or specific fragment thereof, the kit can comprise a labeled compound or agent capable of quantifying this polypeptide. Said agents can be packaged in a suitable container. The kit can further comprise instructions for using the kit to quantify the level of the human pro-EPIL or of the human pro-EPIL transcript.

In certain embodiments of the method of the present invention, the determination of the human pro-EPIL transcripts involves the use of a probe/primer in a polymerase chain reaction (PCR), such as anchor PCR or RACE PCR, or, alternatively, in a ligation chain reaction (LCR) (see, e.g., Landegran et al., 1988, Science 241:23-1080; and Nakazawa et al., 1994, Proc. Natl. Acad. Sci. USA, 91:360-364), or alternatively quantitative real time RT-PCR This method can include the steps of collecting a sample of cells from a patient, isolating nucleic acid (e.g. mRNA) from the cells of the sample, optionally transforming mRNA into corresponding cDNA, contacting the nucleic acid sample with one or more primers which specifically hybridize to the pro-EPIL mRNA or their corresponding cDNA under conditions such that hybridization and amplification of the pro-EPIL mRNA or cDNA occurs, and quantifying the presence of the amplification products. It is anticipated that PCR and/or LCR may be desirable to use as an amplification step in conjunction with any of the techniques used for quantifying nucleic acid detecting.

The methods described herein may be performed, for example, by utilizing prepackaged diagnostic kits comprising at least one probe nucleic acid or set of primer or antibody reagent described herein, which may be conveniently used, e.g., in clinical settings to follow-up or diagnose patients.

In another preferred embodiment, the pro-EPIL peptide, or specific peptide fragment thereof, is detected or quantified by western blot analysis, chromatography, immunoassay or immunohistochemistry.

In a more preferred embodiment, said pro-EPIL peptide, is detected or quantified by ELISA immunoassay or radio immunoassay.

In the method of the present invention, it is preferred that the biological sample obtained from the subject is selected from the group comprising whole blood, blood serum or plasma, tumor biopsy or combinations thereof, blood serum or plasma are more preferred.

In another aspect, the present invention is directed to a kit comprising:
a) an antibody directed specifically against the pro-EPIL peptide; and
b) an antibody directed specifically against at least one of the polypeptide selected from the group consisting of the HCG, its subunit HCGβ and the human AFP polypeptide, preferably:
   a) an antibody directed specifically against the pro-EPIL peptide;
   b) an antibody directed specifically against the HCG or its subunit HCGβ polypeptide; and
   c) an antibody directed specifically against the human AFP polypeptide.

The description also discloses a kit comprising:
a) a set of primers capable of amplifying specifically the pro-EPIL RNA or cDNA; and
b) a set of primers capable of amplifying specifically the RNA or cDNA from the group consisting of the human HCG, its subunit HCGβ and the human alpha-fetoprotein AFP RNA or cDNA.

The present invention also relates to a kit comprising:
a) a set of primers capable of amplifying specifically the pro-EPIL RNA or cDNA;
b) a set of primers capable of amplifying specifically the HCG or its subunit HCGβ RNA or cDNA; and
c) a set of primers capable of amplifying specifically the human AFP RNA or cDNA.

The description discloses a kit comprising:
a) a nucleic probe capable of hybridising specifically with the pro-EPIL RNA; and
b) a nucleic probe capable of hybridising specifically with at least one of the RNA selected from the group consisting of the human gonadotropin HCG, its beta subunit HCGβ and the human alpha-fetoprotein AFP RNA,
preferably:
The present invention also relates to a kit consisting of:
a) a nucleic probe capable of hybridising specifically with the pro-EPIL RNA;
b) a nucleic probe capable of hybridising specifically with the HCG or its subunit HCGβ RNA; and
c) a nucleic probe capable of hybridising specifically with the human AFP RNA.

In the methods or the kits according to the present invention, the antibodies or the probe can be labeled when it is necessary.

Are also comprised in the present invention the kits of the present invention which are suitable for performing the method for detecting and/or classifying a testicular cancer in a subject, to identifying compound of interest or to control the efficiency of an anti-cancer treatment according to the above claimed invention.

One or more reagents necessary to the detection or the quantification of the biomarker may be immobilized onto solid support, such as biochips to form a two-dimension array, for example, a 9 mm x 9 mm array, 12 mm x 12 mm array, and 15 mm x 15 mm array. One or more arrays may be arranged on one biochip, and one or more samples can be tested using one biochip. In some embodiments, the solid support of the biochip comprises a surface selected from the group consisting of a ceramic, a glass, a silica, a quartz, a nylon, a plastic, a polystyrene, a nitrocellulose, and a metal. This type of support and method using biochip (protein or nucleic acid biochip) are well known by the skilled man to perform diagnosing tests.

Thus, also described is a solid-phase nucleic acid molecule array or a solid support comprising:
a) a nucleic acid molecule capable of hybridising specifically with the pro-EPIL RNA; and
b) a nucleic acid molecule capable of hybridising specifically with at least one of the RNA selected from the group consisting of the human gonadotropin HCG, its beta subunit HCGβ and the human alpha-fetoprotein AFP RNA,
fixed to a solid substrate,
preferably described is a solid-phase protein array or solid support comprising:
a) an antibody directed specifically against the pro-EPIL peptide; and
b) an antibody directed specifically against at least one of the polypeptide selected from the group consisting of the HCG, its subunit HCGβ and the human alpha-fetoprotein AFP polypeptide,
fixed to a solid substrate. The present invention also relates to solid phase arrays as claimed in claim 13 and 14.

Finally, the present invention is directed to the use of the expression of the pro-EPIL gene as a biomarker, preferably as a serum (or plasma) or cellular biomarker, for the diagnosing of testicular cancer, particularly of a testicular germ cell tumor.

It is to be understood that while the invention has been described in conjunction with the above embodiments, that the foregoing description and the following examples are intended to illustrate and not limit the scope of the invention. Other aspects, advantages and modifications within the scope of the invention will be apparent to those skilled in the art to which the invention pertains.

### LEGENDS OF THE FIGURES

**Figure 1****:** Schematic representation of the primary structure of pro-EPIL peptide and localization of antibody binding sites recognized by monoclonal antibodies EPIL15 and EPIL02. Amino acid residues are indicated by one-letter code.
**Figure 2****:** Serum levels of PEP in healthy male subjects and in patients with Seminomatous and non-seminomatous testicular germ-cell tumors. O, 10 cases; ●, 1 case.
**Figures 3A-3B****:** Representative examples of serum levels of PEP and HCGβ in two patients with non-seminomatous testicular germ-cell tumors: patient with a mixed tumor composed of embryonal carcinoma and seminoma. (A) Patient with a mixed tumor composed of choriocarcinoma, yolk sac tumor, teratoma and seminoma.
**Figures 4A-4F****:** Immunohistochemistry staining of PEP (Figs. 4A, 4C, 4E) and HCGβ (Figs; 4B, 4D, 4F) in a mixed tumor composed of embryonal carcinoma, teratoma and choriocarcinoma (A, B) and in a mixed tumor composed of embryonal carcinoma, yolk sac tumor and seminoma with syncytiotrophoblastic cells (Figs. 4C to 4F). Photos were taken at either original 10x (Figs. 4A to 4D) or original 20x (Figs. 4E, 4F) magnification.

### EXAMPLES

### 1) PATIENTS AND METHODS

### a) Study population and sample collection

All serum samples were selected from our institutional review-board-approved repository. The samples were all collected, processed and stored at -20°C in a similar fashion. Serum samples from 52 patients with testicular germ-cell cancer and followed for up to 10 years, and from 104 healthy male subjects, were studied after receiving their informed consent. Patient demographics and histology are presented in Table 1.

**Table 1: Patient demographics and histology of testicular germ-cell tumors**

| | Patients | | | | Patients Number |
|---|---|---|---|---|---|
| Demographics | Age in years range | | | 34.8 + 10.9 17- 69 | 52 |
| | Serum level measurement | | | | |
| | | | | before orchidectomy | 9 |
| | | | | after orchidectomy | 43 |
| | Delay between orchidectomy and serum measurement | | | | |
| | Days | | | 59 + 66.2 | |
| | Range | | | 1 - 303 | |
| | Tumors | | | | |
| Histology | Seminomatous | | | | **25** |
| | | Seminoma | | | 21 |
| | | Seminoma with syncytiotrophoblastic cells | | | 4 |
| | | | | | |
| | Non-seminomatous | | | | **27** |
| | | | | | |
| | | Pure forms | | | 5 |
| | | | Embryonal carcinoma | | 4 |
| | | | Yolk sac | | 1 |
| | | | | | |
| | | Mixed forms | | | 22 |
| | | | Embryonal carcinoma + seminoma | | 5 |
| | | | Embryonal carcinoma + yolk sac | | 2 |
| | | | Embryonal carcinoma + teratoma | | 1 |
| | | | Teratoma + seminoma | | 1 |
| | | | Choriocarcinoma +seminoma | | 1 |
| | | | Other (more than two histological types) | | 12 |

Clinical data, including stage at diagnosis, histology, treatment modalities and outcome, were available for each patient whose serum was used in the study. Moreover, tissue specimens from two patients with testicular cancer were selected from the established tumor library of our comprehensive cancer Center, in accordance with protocols that had been approved by the local ethical committee. Tissue specimens were fixed in 10 % neutral-buffered formalin and paraffin-embedded using standard procedures. Serial sections (5 µm) were cut for histology and immunohistochemistry.

PEP enzyme-linked immunosorbent assay and immunoassays for detection of HCGβ and AFP.

PEP serum levels were measured in sera using an ELISA based on monoclonal antibody (mAb) EPIL15 (kindly supplied by Prof. Bidart, Institut Gustave-Roussy, Villejuif, France) directed to the EPIL C-chain 98-108 region as previously described. ¹¹This antibody serves as capture antibody on a solid phase support, while biotinylated mAb EPIL02 (kindly supplied by Prof. Bidart, Institut Gustave-Roussy, Villejuif, France) directed to the EPIL A-chain 125-137 region is used as tracer. The standard curve was constructed with a peptide spanning the 76-139 portion of pro-EPIL used at increasing concentrations ranging from 0.7 ng/mL to 100 ng/mL. Linearity in a range of 0.7 ng/mL to 100 ng/mL was consistently shown in between and within-run assays. The coefficients of variation interassay (n = 10) and intra-assay (n = 33) were 12 % and 13.3 %, respectively at 6.6 ng/mL and 6 % and 7.5 %, respectively at 50 ng/mL. The sensitivity of this ELISA is 1 ng/mL.

A commercial IRMA (ELSA-FβHCG, CIS bio international, Gif-sur-Yvette, France) based on highly specific monoclonal antibody mAb FBT11 was used to measure HCGβ. The immunoassay for HCGβ displays a sensitivity of 100 pg/mL.¹² AFP serum levels were measured by a commercial immunoassay (BRAHMS-AFP Kryptor, Hennigsdorf, Germany) based on highly specific mAb AF01.¹³ This assay displays a sensitivity of 0.23 ng/mL.

### b) Histology and immunohistochemistry

Histology of each specimen was determined by visual examination of tissue sections stained with hematoxylin, eosin and safran. Immunostaining was performed using an automated immunostainer (Dako Autostainer) and a streptavidine biotine peroxidase method (Dako Real^{™} Detection System LSAB+, Dako SAS, Trappes, France) with mAb EPIL15 directed to PEP, polyclonal antibody directed to HCG (Dako SAS, Trappes, France) and polyclonal antibody directed to AFP (Dako SAS, Trappes, France).

### c) Statistical analysis

Statistical analyses were performed using GraphPad Prism version 4.0. Correlations were studied with non-parametric (Spearman) tests.

### 2) RESULTS

### a) Serum levels of PEP, HCGβ and AFP at first determination

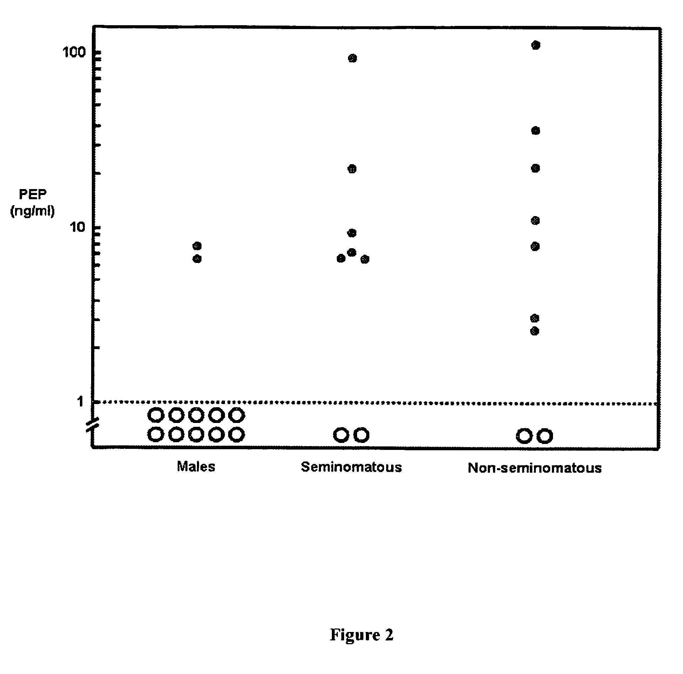

Serum levels of PEP, HCGβ and AFP were measured in 25 patients with seminomatous testicular germ-cell tumors and in 27 patients with non-seminomatous tumors (Table 1). Serum samples were collected before orchidectomy in 9 patients and after orchidectomy in 43 patients. In parallel, serum levels of PEP were measured in 104 healthy male subjects. PEP serum values are shown in Figure 2. In healthy subjects, only 2 out of 104 (1.9 %) had serum values higher than 1 ng/mL, while 13 out 52 patients (25 %) displayed serum PEP levels higher than 1 ng/mL. In patients with seminomatous and non-seminomatous germ cell tumors, PEP was present in 24 % and 25 % of serum samples at first determination, respectively. In this series of 52 patients, serum levels of HCGβ was detected in 17 patients (32.6 %) and AFP was elevated in the sera of 12 patients (23 %). Data analysis with non-parametric (Spearman) tests showed that PEP serum values were not correlated with serum levels of either HCGβ (r = 0.0076) or AFP (r = -0.0548). Indeed, one and/or two of the latter biomarkers were present in 44.2% of patients, while PEP, HCGβ and/or AFP were detected in the sera of 59.6 % of patients with seminomatous or non-seminomatous testicular cancers (Table 2).

**Table 2: Patients with elevated serum levels of PEP (>1 ng/ml), HCGβ (>0.1 ng/ml) and/or AFP (>10 ng/ml)**

| Histology | Patient Number | Serum biomarker(s) | | | | |
|---|---|---|---|---|---|---|
| | | PEP | HCGβ | AFP | Free HCGβ or AFP | PEP or HCGβ or AFP |
| Seminomatous | 25 | 6 | 9 | 0 | 10 | 13 |
| Non-seminomatous | 27 | 7 | 8 | 12 | 13 | 17 |
| Total | 52 | 13 (25 %) | 17 (32.6 %) | 12 (23 %) | 23 (44.2 %) | 31 (59.6 %) |

### b) Serial measurements of PEP and HCGβ serum levels

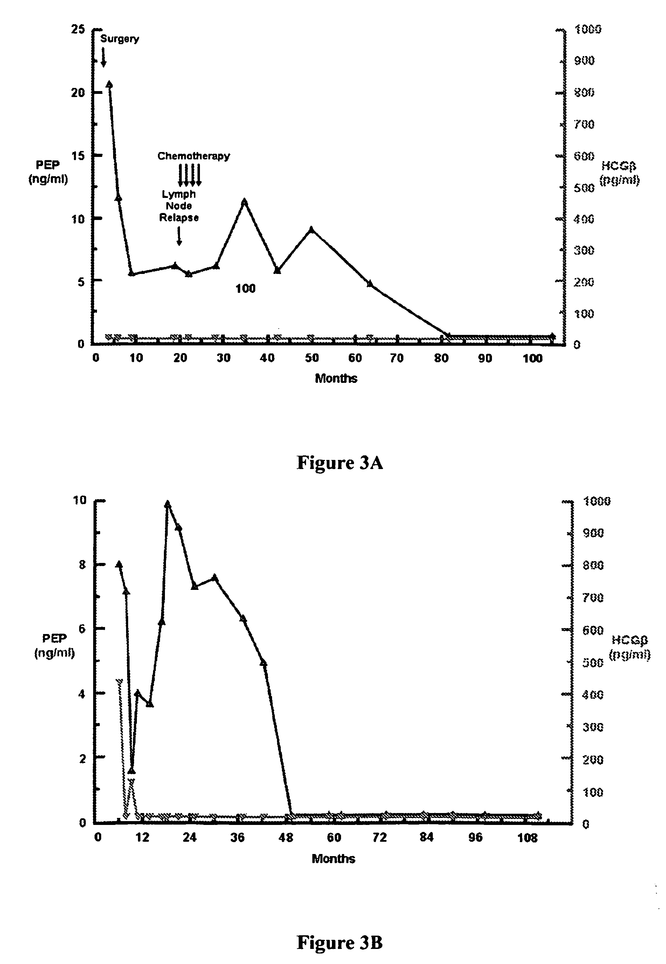

Serial determination of PEP and HCGβ was carried out in two patients followed up for at least four years and taken as representative (Figures 3A and 3B). One patient had elevated serum values of PEP for a period of 42 months after orchidectomy, while neither HCGβ nor AFP was ever detected (Figure 3A). However, in this patient, the initial serum sample available for this study had been drawn 3 months after surgery. It is noteworthy that, after an initial decrease in PEP values following orchidectomy, a rise in serum PEP levels and lymph node relapse occurred concurrently in this patient, who had a mixed form of a non-seminomatous germ cell tumor composed of embryonal carcinoma and seminoma. The other representative patient had measurable levels of PEP, HCGβ and AFP (116 UI/mL) on the initial serum sample drawn prior to orchidectomy. After surgery, AFP levels were consistently below the upper limit of the usual values found in healthy subjects (< 5 ng/mL).¹³ A low level of HCGβ (0.122 pg/mL) was still detected 3 months after surgery. In striking contrast, high levels of PEP were detected up to 3 years after surgery for this tumor, which was a non-seminomatous germ cell tumor comprising choriocarcinoma, yolk sac tumor, teratoma and seminoma.

### c) Expression of PEP at the cellular level

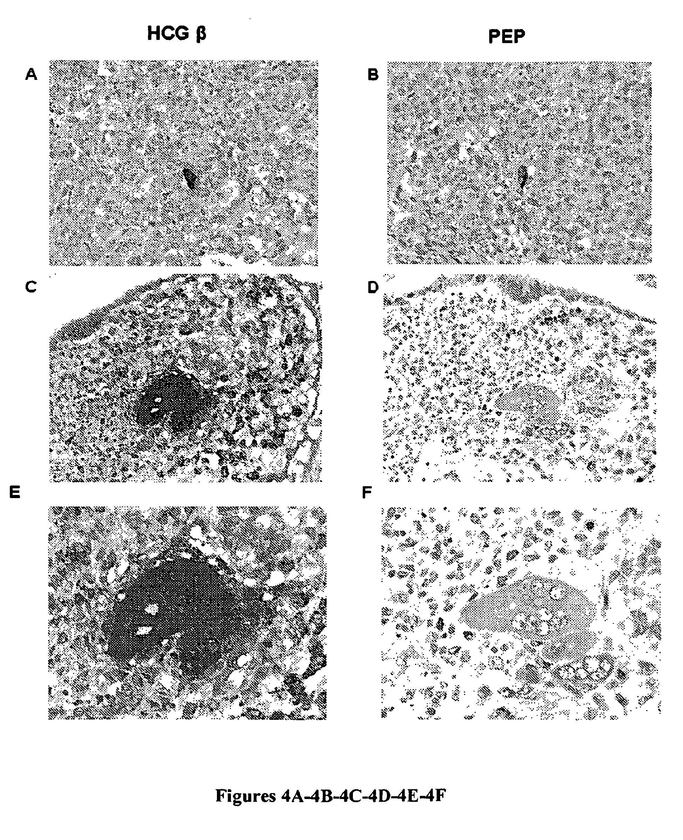

In order to identify PEP-producing cells, immunohistochemistry studies were performed on histological sections of two testis tumors excreting measurable serum levels of PEP. One tumor was a mixed form of non-seminomatous germ cell tumor composed of embryonal carcinoma, teratoma and choriocarcinoma (Figures 4A and 4B) and the other was a mixed form of a non-seminomatous germ cell tumor composed of embryonal carcinoma, yolk sac tumor and seminoma with syncytiotrophoblastic cells (Figures 4C to 4F). On tissue sections studied, AFP-producing cells were not present. In contrast, HCGβ-producing cells were strongly stained with mAb directed to HCGβ. In these tumors, PEP staining was detected with mAb EPIL¹⁵ in multinucleated (syncytiotrophoblastic) cells, while, in one tumor, staining was also detected in mononucleated cells (Figures 4E and 4F). In the latter tumor, the intensity of staining was stronger in mononucleated cells compared to multinucleated cells.

### 3) CONCLUSION

Testicular cancer is the most common solid tumor in young men between the ages of 15 and 34, and 95% of these cancers are germ-cell tumors, a term that indicates their origin in primordial germ-cells. 1 Sensitive tumor markers and effective treatment modalities have transformed the prognosis for these cancers and they are now highly curable. However, several aspects of testicular germ-cell tumors remain challenging: The incidence of these cancers is rising and a delay in diagnosis may still be too long for some patients, thereby affecting their prognosis. Although this prognosis is considered good in a large majority of patients, a group with poor prognosis remains. On a broader perspective, a recently observed slowdown in the decline in mortality rates is cause for concern. Another challenge is the need to minimize long-term toxic effects of therapy without jeopardizing effectiveness. While the three biomarkers, HCG, HCGβ and AFP, effectively contribute to management of testicular cancer, novel biomarkers would be helpful in improving this management. Among the three latter biomarkers, it is noteworthy that, in terms of expression, both HCG and HCGβ share similarities with a distinctive group of antigens called cancer/testis (C/T) antigens. In order for a protein to be designated a C/T antigen, it must be expressed in tumors as well as in testis and/or the placenta, but must not be expressed in more than two non-germ-line normal tissues. When non-germ-line normal tissue expression is detected, this is usually at only a fraction of the level detected in the testis.¹⁴ Like HCGβ, tumor antigen MZ2-E, which was the first C/T antigen described, along with a growing number of C/T antigens (more than 40) which have now been identified, are highly expressed in placenta and tumors.¹⁵ Likewise, PEP is expressed in placenta and tumors: it was reported that c-erbB-2-positive breast cancer cells with high invasion potential express and secrete PEP.¹⁶ Moreover, as expected from a C/T antigen, PEP expression was detected in only one non-germ-line normal tissue and this expression was faint. Thus, PEP expression displays most of the hallmarks of cancer/testis (CT) antigens, and this study was designed to determine whether this antigen is also a biomarker of testicular cancers.

PEP is detected in about half of males presenting with either seminomatous (24 %) or non-seminomatous (25.9 %) testicular tumors (Table 2). In contrast, measurable serum levels were found in only 2 out of 104 (1.9 %) healthy male subjects. It is not uncommon that tumor markers, including HCG and HCGβ, are present in a limited number of healthy male subjects. Indeed, sera from men and non-pregnant women contain low levels of HCG and HCGβ that can be detected by sensitive assays; it is likely that detection of PEP in fewer than 2 % of healthy males is related to the sensitivity of the immunoassay used for measuring PEP. ^{17,18} More importantly, it was striking that 8 out of 13 (61.5 %) PEP-producing tumors did not secrete measurable levels of either HCGβ or AFP. Indeed, in this series of 52 patients with germ-cell testicular cancers, 44.2 % of patients had measurable serum levels of HCGβ and/or AFP, while HCGβ, AFP and/or PEP levels were elevated in sera of 59.6 % of patients. It is likely that these percentages were underestimated, since most serum samples were collected 1 to 303 days after orchidectomy: in that series, 48.1 % of patients with non-seminomatous tumors had elevated serum levels of HCGβ and/or AFP, while it had been previously observed that one or both of these markers are expressed in about three-fourths of non-seminomas.²

Another unexpected observation was the persistence of measurable serum levels of PEP in certain patients for up to 42 months after the end of treatment. It is likely that the persistence of measurable serum levels of PEP indicates the presence of remaining tumor cells. Surgery of residual masses after chemotherapy in patients with testicular cancer show that 45 % and 10 % of residual masses contain teratomas or active disease, respectively.¹⁹ Moreover, it was reported that patients with a variety of primary germ-cell tumors in the testis and who are treated with radiation therapy and/or chemotherapy in addition to surgery may develop mature teratomas at differing anatomical sites.²⁰ Interestingly, one patient with a non-seminomatous germ-cell tumor composed of embryonal carcinoma and seminoma displayed a rise in PEP serum levels prior to lymph node relapse, followed by persistent serum levels of PEP after completion of chemotherapy (Figure 3A). Another patient with persistent levels of PEP did not receive radiation therapy or chemotherapy (Figure 3B). That patient had a non-seminomatous germ-cell tumor composed of choriocarcinoma, yolk sac tumor, teratoma and seminoma. The types of tumor cells that continue to produce PEP thus remain to be determined. Indeed, testicular cancers with a wide variety of histological types secrete PEP. In our series, PEP was excreted by 6 pure seminomas without syncytiotophoblast cells, 1 embryonal carcinoma and 6 mixed tumors containing two or three differing histological types (1 embryonal carcinoma and seminoma, 1 choriocarcinoma and seminoma, 1 teratoma and seminoma, 1 embryonal carcinoma, teratoma and choriocarcinoma, 1 embryonal carcinoma, yolk sac and seminoma with syntytiotrophoblastic cells and I choriocarcinoma, yolk sac tumor, teratoma and seminoma). In order to characterize the histological type of expressing cell, immunohistochemistry staining was performed on two mixed tumors with antibodies directed to HCGβ, AFP and PEP: one testis tumor contained embryonal carcinoma, teratoma and choriocarcinoma (Figures 4A and 4B) and the other tumor was a mixed form of non-seminomatous germ cell tumor composed of embryonal carcinoma, yolk sac tumor and seminoma with syncytiotrophoblastic cells (Figures 4C to 4F). On these tissue sections, multinucleated cells appeared to produce both HCGβ and PEP, with the same (Figures 4A and 4B) or differing (Figures 4C and 4D) staining intensities. In mononucleated cells, these two proteins may be produced by the same or by distinct mononucleated cells.

Taken together, observations at the serum and cellular levels show that, in contrast to normal placenta in which biosynthesis of HCGβ and PEP may be regulated by common pathways, in germ-cell testicular tumors, their production might be regulated by differing pathways.¹⁰

Finally, PEP is a serum biomarker which provides a complement of information on germ-cell testicular cancers. PEP may be the only biomarker present in sera of seminomatous and non-seminomatous testicular tumors, indicating the presence of tumor cells previously undetected by other serum biomarkers at clinical diagnosis. PEP may also indicate the presence of residual tumor cells still present several years after the end of treatment.

### REFERENCES

1. Bosl GJ, Motzer RJ: Testicular germ-cell cancer. N Engl J. Med. 337:242-53, 1997.
2. Horwich A, Huddart R, Dearnaley D: Markers and management of germ-cell tumors of the testes. Lancet 352:1535-8, 1998.
3. Levi F, La Vecchia C, Boyle P, et al: Western and eastern European trends in testicular cancer mortality. Lancet 357:1853-4, 2001.
4. Horwich A, Shipley J, Huddart R: Testicular germ-cell cancer. Lancet 367:754-65,2006.
5. Dieckmann KP: Diagnostic delay in testicular cancer: an analytic chimaera or a worthy goal. Eur Urol 52:1566-8, 2007.
6. Huyghe E, Muller A, Mieusset R, et al: Impact of diagnostic delay in testis cancer: results of a large population-based study. Eur Urol 52:1710-6, 2007.
7. Huyghe E, Matsuda T, Thonneau P: Increasing incidence of testicular cancer worldwide: a review. J. Urol. 170:5-11, 2003.
8. Chassin D, Laurent A, Janneau JL, et al: Cloning of a new member of the insulin gene superfamily (INSL4) expressed in human placenta. Genomics 29:465-70, 1995.
9. Bellet D, Lavaissiere L, Mock P, et al: Identification of pro-EPIL and EPIL peptides translated from insulin-like 4 (INSL4) mRNA in human placenta. J Clin Endocrinol Metab 82:3169-72, 1997.
10. Mock P, Frydman R, Bellet D, et al: Pro-EPIL forms are present in amniotic fluid and maternal serum during normal pregnancy. J Clin Endocrinol Metab 84:2253-6, 1999.
11. Bruni L, Luisi S, Ferretti C, et al: Changes in the maternal serum concentration of proearly placenta insulin-like growth factor peptides in normal vs abnormal pregnancy. Am. J. Obstet. Gynecol. 197:606 el-4, 2007.
12. Marcillac I, Troalen F, Bidart JM, et al: Free human chorionic gonadotropin beta subunit in gonadal and nongonadal neoplasms. Cancer Res 52:3901-7, 1992.
13. Bellet DH, Wands JR, Isselbacher KJ, et al: Serum alpha-fetoprotein levels in human disease: perspective from a highly specific monoclonal radioimmunoassay. Proc Natl Acad Sci U S A, 81:3869-73, 1984.
14. Scanlan MJ, Simpson AJ, Old LJ: The cancer/testis genes: review, standardization, and commentary. Cancer Immun. 4:1, 2004.
15. van der Bruggen P, Traversari C, Chomez P, et al: A gene encoding an antigen recognized by cytolytic T lymphocytes on a human melanoma. Science 254:1643-7, 1991.
16. Brandt B, Roetger A, Bidart JM, et al: Early placenta insulin-like growth factor (pro-EPIL) is overexpressed and secreted by c-erbB-2-positive cells with high invasion potential. Cancer Res 62:1020-4, 2002.
17. Stenman UH, Tiitinen A, Alfthan H, et al: The classification, functions and clinical use of different isoforms of HCG. Hum Reprod Update 12:769-84, 2006.
18. Alfthan H, Haglund C, Dabek J, et al: Concentrations of human choriogonadotropin, its beta-subunit, and the core fragment of the beta-subunit in serum and urine of men and nonpregnant women. Clin Chem 38:1981-7, 1992.
19. Flechon A, Rivoire M, Berger N: [Surgery of residual masses after chemotherapy in patients with testicular cancer]. Rev Prat 57:389-98, 2007.
20. Hong WK, Wittes RE, Hajdu ST, et al: The evolution of mature teratoma from malignant testicular tumors. Cancer 40:2987-92, 1977.

## Claims

1. An in vitro method for detecting and/or classifying a testicular cancer in a subject, comprising the step of determining the expression level of the gene encoding the pro-EPIL peptide in a biological sample isolated from said subject wherein overexpression of said gene encoding the pro-EPIL peptide is indicative of the presence of a testicular cancer and/or class of testicular germ cell tumor, preferably a seminomatous or non-seminomatous testicular cancer and/or class of testicular germ cell tumor.

2. The method of claim 1, wherein said method further comprises a step of determining the expression level of at least one gene selected from the group of gene consisting of the genes encoding the human gonadotropin HCG, its beta subunit HCGβ and the human alpha-fetoprotein AFP in a biological sample isolated from said subject wherein overexpression of at least one of said gene encoding the encoding the HCG, its beta subunit HCGβ or the human AFP is indicative of the presence of a testicular cancer and/or class of testicular germ cell tumor.

3. The method of claim 2, wherein said method further comprises a step of determining the expression level of the genes encoding the HCG, or its subunit HCGβ, and the human AFP in a biological sample isolated from said subject wherein overexpression of at least one of said gene encoding the HCG, or its subunit HCGβ, or the human AFP is indicative of the presence of a testicular cancer and/or class of testicular germ cell tumor.

4. The method of claim 3, wherein the presence of an overexpression of the gene encoding the pro-EPIL peptide, an overexpression of the gene encoding the HCGβ and an overexpression of the gene encoding the alpha-fetoprotein AFP in a biological sample isolated from said is indicative of the presence of a non-seminatous testicular cancer and/or class of testicular germ cell tumor.

5. A method for identifying a compound candidate for a pharmacological agent useful in the treatment of testicular cancer, particularly a testicular germ cell tumor, comprising the step of:
a) contacting a non-human mammal subject presenting a testicular cancer, particularly a testicular germ cell tumor, with a candidate pharmacological agent;
b) determining the expression level of the gene encoding the pro-EPIL peptide in a biological sample isolated from said subject;
c) optionally, further determining the expression level of at least one gene selected from the group of gene consisting of the genes encoding the HCG, its subunit HCGβ and the human AFP in a biological sample isolated from said subject,
wherein a decrease in the test amount of expression of the gene encoding the pro-EPIL peptide, and optionally, a decrease of the expression of the gene determined in step c), indicates that the candidate pharmacological agent is a potential compound for a pharmacological agent useful in the treatment of testicular cancer, particularly a testicular germ cell tumor.

6. A method for evaluating the effect in a subject of a treatment for testicular cancer, particularly a testicular germ cell tumor, comprising the step of:
a) determining the expression level of the gene encoding the pro-EPIL peptide in a biological sample isolated from said subject;
b) determining a second expression level of the gene encoding the pro-EPIL peptide in a biological sample isolated from said subject;
c) comparing the first and second amounts of expression level of the gene encoding the pro-EPIL peptide in a biological sample isolated from said subject;
wherein a decrease of the expression level of the gene encoding the pro-EPIL peptide in the second biological sample isolated from said subject indicates the regression of said testicular cancer, particularly a testicular germ cell tumor.

7. The method or any one of claims 1 to 6, wherein the expression level is determined by detecting the presence, absence or level of mRNA transcribed from said gene or of polypeptide encoded by said gene, or specific fragment thereof.

8. The method of claim 7, wherein said polypeptide is detected or quantified by western blot analysis, chromatography, immunoassay or immunohistochemistry.

9. The method of any of claims 1 to 8, wherein the biological sample obtained from the subject is selected from the group comprising whole blood, blood serum or plasma, tumor biopsy or combinations thereof.

10. A kit for detecting and/or classifying a testicular cancer, particularly a testicular germ cell tumor, comprising:
a) an antibody directed specifically against the pro-EPIL peptide;
b) an antibody directed specifically against the HCG or its submit HCGβ polypeptide; and
c) an antibody directed specifically against the human AFP polypeptide.

11. A kit for detecting and/or classifying a testicular cancer, particularly a testicular germ cell tumor, comprising:
a) a set of primers capable of amplifying specifically the Pro-EPIL RNA or cDNA; and
b) a set of primers capable of amplifying specifically the RNA or cDNA from the group consisting of the HCG, its subunit HCGβ; and
c) a set of primers capable of amplifying the human alpha-fetoprotein AFP RNA or cDNA.

12. A kit for detecting and/or classifying a testicular cancer, particularly a testicular germ cell tumor, consisting of:
a) a nucleic probe capable of hybridising specifically with the Pro-EPIL RNA;
b) a nucleic probe capable of hybridising specifically with the human gonadotropin HCG or its beta subunit HCGβ RNA; and
c) a nucleic probe capable of hybridising specifically with the human alpha-fetoprotein AFP RNA.

13. A solid-phase nucleic acid molecule array for detecting and/or classifying a testicular cancer, particularly a testicular germ cell tumor, consisting of:
a) a nucleic acid molecule capable of hybridising specifically with the pro-EPIL RNA;
b) a nucleic acid molecule capable of hybridising specifically with the human gonadotropin HCG or its beta subunit HCGβ RNA; and
c) a nucleic acid molecule capable of hybridising specifically with the human alpha-fetoprotein AFP RNA.

14. A solid-phase protein microarray for detecting and/or classifying a testicular cancer, particularly a testicular germ cell tumor, comprising:
a) an antibody directed specifically against the pro-EPIL peptide;
b) an antibody directed specifically against the HCG or its subunit HCGβ polypeptide; and
c) an antibody directed specifically against the human AFP polypeptide,
fixed to a solid substrate.

15. Use of the expression of the pro-EPIL gene as a biomarker for the diagnosing of testicular cancer, particularly a testicular germ cell tumor.

## Patentansprüche

1. In-Vitro-Verfahren zum Nachweis und/oder zur Klassifizierung von Hodenkrebs in einem Subjekt, umfassend den Schritt des Bestimmens des Expressionsniveaus des Gens, welches das Pro-EPIL-Peptid kodiert, in einer von dem betreffenden Subjekt genommenen biologischen Probe, wobei die Überexpression des Gens, welches das Pro-EPIL-Peptid kodiert, auf das Vorliegen von Hodenkrebs und/oder die Klasse eines testikulären Keimzelltumors, bevorzugt von seminomatösem oder nicht-seminomatösem Hodenkrebs und/oder die Klasse eines testikulären Keimzelltumors, hinweist.

2. Verfahren gemäß Anspruch 1, wobei das Verfahren ferner einen Schritt des Bestimmens des Expressionsniveaus wenigstens eines Gens, ausgewählt aus der Gruppe bestehend aus den Genen, welche das humane Gonadotropin HCG, dessen Beta-Untereinheit HCGβ und das humane Alpha-Fetoprotein AFP kodieren, in einer von dem betreffenden Subjekt genommenen biologischen Probe umfasst, wobei die Überexpression wenigstens eines der Gene, welche das HCG, dessen Beta-Untereinheit HCGβ oder das humane AFP kodieren, auf das Vorliegen von Hodenkrebs und/oder die Klasse eines testikulären Keimzelltumors hinweist.

3. Verfahren gemäß Anspruch 2, wobei das Verfahren ferner einen Schritt des Bestimmens des Expressionsniveaus der Gene, welche das HCG oder dessen Untereinheit HCGβ und das humane AFP kodieren, in einer von dem betreffenden Subjekt genommenen biologischen Probe umfasst, wobei die Überexpression wenigstens eines der Gene, welche das HCG oder dessen Untereinheit HCGβ oder das humane AFP kodieren, auf das Vorliegen von Hodenkrebs und/oder die Klasse eines testikulären Keimzelltumors hinweist.

4. Verfahren gemäß Anspruch 3, wobei das Vorliegen einer Überexpression des Gens, welches das Pro-EPIL-Peptid kodiert, eine Überexpression des Gens, welches das HCGβ kodiert, und eine Überexpression des Gens, welches das Alpha-Fetoprotein AFP kodiert, in einer von dem betreffenden Subjekt genommenen biologischen Probe auf das Vorliegen eines nicht-seminomatösen Hodenkrebses und/oder die Klasse eines testikulären Keimzelltumors hinweist.

5. Verfahren zur Identifizierung einer Kandidaten-Verbindung für ein pharmakologisches Agens, welches zur Behandlung von Hodenkrebs, insbesondere eines testikulären Keimzelltumors, geeignet ist, umfassend den Schritt:
a) In-Kontakt-Bringen eines nicht-menschlichen Säugetiers, welches einen Hodenkrebs, insbesondere einen testikulären Keimzelltumor, aufweist, mit einem Kandidaten für ein pharmakologisches Agens;
b) Bestimmen des Expressionsniveaus des Gens, welches das Pro-EPIL-Peptid kodiert, in einer von dem betreffenden Subjekt genommenen biologischen Probe;
c) optional, außerdem Bestimmen des Expressionsniveaus wenigstens eines Gens, ausgewählt aus der Gruppe bestehend aus den Genen, welche das HCG, dessen Untereinheit HCGβ und das humane AFP kodieren, in einer von dem betreffenden Subjekt genommenen biologischen Probe,
wobei eine Abnahme der Expressionsmenge des Gens, welches das Pro-EPIL-Peptid kodiert, und, optional, eine Abnahme der Expression des in Schritt c) bestimmten Gens darauf hinweist, dass der Kandidat für das pharmakologische Agens eine potentielle Verbindung für ein pharmakologisches Agens darstellt, welches für die Behandlung von Hodenkrebs, insbesondere eines testikulären Keimzelltumors, geeignet ist.

6. Verfahren zur Bewertung der Wirkung einer Hodenkrebs-Behandiung, insbesondere einer Behandlung eines testikulären Keimzelltumors, in einem Subjekt, umfassend die Schritte:
a) Bestimmen des Expressionsniveaus des Gens, welches das Pro-EPIL-Peptid kodiert, in einer von dem betreffenden Subjekt genommenen biologischen Probe;
b) Bestimmen eines zweiten Expressionsniveaus des Gens, welches das Pro-EPIL-Peptid kodiert, in einer von dem betreffenden Subjekt genommenen biologischen Probe;
c) Vergleichen der ersten und zweiten Expressionsniveaus/- mengen des Gens, welches das Pro-EPIL-Peptid kodiert, in einer von dem betreffenden Subjekt genommenen biologischen Probe,
wobei eine Abnahme des Expressionsniveaus des Gens, welches das Pro-EPIL-Peptid kodiert, in der zweiten biologischen Probe, die aus dem betreffenden Subjekt isoliert wurde, auf eine Rückbildung des Hodenkrebses, insbesondere eines testikulären Keimzelltumors, hinweist.

7. Verfahren gemäß irgendeinem der Ansprüche 1 bis 6, wobei das Expressionsniveau durch Nachweis der Anwesenheit, der Abwesenheit oder des Niveaus der mRNA, die von dem betreffenden Gen transkribiert wurde, oder eines von dem Gen kodierten Polypeptids oder eines spezifischen Fragments davon bestimmt wird.

8. Verfahren gemäß Anspruch 7, wobei das Polypeptid durch Western-Blot-Analyse, Chromatographie, Immunassay oder Immunhistochemie detektiert oder quantifiziert wird.

9. Verfahren gemäß irgendeinem der Ansprüche 1 bis 8, wobei die von dem Subjekt erhaltene biologische Probe ausgewählt ist aus der Gruppe bestehend aus Gesamtblut, Blutserum oder Plasma, Tumorbiopsie oder Kombinationen davon.

10. Kit zum Nachweis und/oder zur Klassifizierung von Hodenkrebs, insbesondere eines testikulären Keimzelltumors, umfassend:
a) einen Antikörper, der spezifisch gegen das Pro-EPIL-Peptid gerichtet ist;
b) einen Antikörper, der spezifisch gegen das HCG oder dessen HCGβ-Polypeptid-Untereinheit gerichtet ist; und
c) einen Antikörper, der spezifisch gegen das humane AFP-Polypeptid gerichtet ist.

11. Kit zum Nachweis und/oder zur Klassifizierung von Hodenkrebs, insbesondere eines testikulären Keimzelltumors, umfassend:
a) einen Primer-Satz, der zur spezifischen Amplifikation der pro-EPIL-RNA oder -cDNA geeignet ist;
b) einen Primer-Satz, der zur spezifischen Amplifikation der RNA oder cDNA von HCG oder dessen Untereinheit HCGβ geeignet ist; und
c) einen Primer-Satz, der zur Amplifikation der humanen Alpha-Fetoprotein AFP-RNA oder -cDNA geeignet ist.

12. Kit zum Nachweis und/oder zur Klassifizierung von Hodenkrebs, insbesondere eines testikulären Keimzelltumors, bestehend aus:
a) einer Nukleinsonde, die spezifisch mit der pro-EPIL-RNA hybridisieren kann;
b) einer Nukleinsonde, die spezifisch mit der RNA des humanen Gonadotropins HCG oder dessen Beta-Untereinheit HCGβ hybridisieren kann; und
c) einer Nukleinsonde, die spezifisch mit der RNA des humanen Alpha-Fetoproteins AFP hybridisieren kann.

13. Festphasen-Nukleinsäuremolekül-Array zum Nachweis und/oder zur Klassifizierung von Hodenkrebs, insbesondere eines testikulären Keimzelltumors, bestehend aus:
a) einem Nukleinsäuremolekül, das spezifisch mit der pro-EPIL-RNA hybridisieren kann;
b) einem Nukleinsäuremolekül, das spezifisch mit der RNA des humanen Gonadotropins HCG oder dessen Beta-Untereinheit HCGβ hybridisieren kann; und
c) einem Nukleinsäuremolekül, das spezifisch mit der RNA des humanen Alpha-Fetoproteins AFP hybridisieren kann.

14. Festphasen-Protein-Mikroarray zum Nachweis und/oder zur Klassifizierung von Hodenkrebs, insbesondere eines testikulären Keimzelltumors, umfassend:
a) einen Antikörper, der spezifisch gegen das Pro-EPIL-Peptid gerichtet ist;
b) einen Antikörper, der spezifisch gegen das HCG oder dessen HCGβ-Polypeptid-Untereinheit gerichtet ist; und
c) einen Antikörper, der spezifisch gegen das humane AFP-Polypeptid gerichtet ist,
fixiert an ein Festkörpersubstrat.

15. Verwendung der Expression des pro-EPIL-Gens als Biomarker zur Diagnose von Hodenkrebs, insbesondere eines testikulären Keimzelltumors.

## Revendications

1. Procédé *in vitro* pour détecter et/ou classer un cancer du testicule chez un sujet, comprenant l'étape consistant à déterminer le niveau d'expression du gène encodant le peptide pro-EPIL dans un échantillon biologique isolé à partir dudit sujet **caractérisé en ce que** la surexpression dudit gène encodant le peptide pro-EPIL indique la présence d'un cancer du testicule et/ou d'une classe de tumeurs germinales du testicule, de préférence d'un cancer séminomateux ou non séminomateux du testicule et/ou d'une classe de tumeurs germinales séminomateuses ou non séminomateuses du testicule.

2. Procédé selon la revendication 1, **caractérisé en ce que** ledit procédé comprend en outre une étape consistant à déterminer le niveau d'expression d'au moins un gène choisi dans le groupe de gènes consistant en les gènes encodant la gonadotrophine humaine HCG, sa sous-unité bêta HCGβ et l'alpha-foetoprotéine humaine AFP dans un échantillon biologique isolé à partir dudit sujet où la surexpression d'au moins un desdits gènes encodant la HCG, sa sous-unité HCGβ ou l'AFP humaine indique la présence d'un cancer du testicule et/ou d'une classe de tumeurs germinales du testicule.

3. Procédé selon la revendication 2, **caractérisé en ce que** ledit procédé comprend en outre une étape consistant à déterminer le niveau d'expression des gènes encodant la HCG ou sa sous-unité HCGβ et l'AFP humaine dans un échantillon biologique isolé à partir dudit sujet où la surexpression d'au moins un desdits gènes encodant la HCG ou sa sous-unité HCGβ ou l'AFP humaine indique la présence d'un cancer du testicule et/ou d'une classe de tumeurs germinales du testicule.

4. Procédé selon la revendication 3, **caractérisé en ce que** la présence d'une surexpression du gène encodant le peptide pro-EPIL, d'une surexpression du gène encodant la HCGβ et d'une surexpression du gène encodant l'alpha-foetoprotéine humaine AFP dans un échantillon biologique isolé à partir dudit sujet indique la présence d'un cancer non séminomateux du testicule et/ou d'une classe de tumeurs germinales non séminomateuses du testicule.

5. Procédé pour identifier un composé candidat pour un agent pharmacologique utile dans le traitement du cancer du testicule, notamment d'une tumeur germinale du testicule, comprenant les étapes consistant à :
a) mettre en contact un sujet de type mammifère non humain présentant un cancer du testicule, notamment une tumeur germinale du testicule, avec un agent pharmacologique candidat ;
b) déterminer le niveau d'expression du gène encodant le peptide pro-EPIL dans un échantillon biologique isolé à partir dudit sujet ;
c) facultativement, déterminer en outre le niveau d'expression d'au moins un gène choisi dans le groupe de gènes consistant en les gènes encodant la HCG, sa sous-unité HCGβ et l'AFP humaine dans un échantillon biologique isolé à partir dudit sujet,
**caractérisé en ce qu'**une diminution du taux testé d'expression du gène encodant le peptide pro-EPIL et, facultativement, une diminution de l'expression du gène déterminée à l'étape c) indique que l'agent pharmacologique candidat est un composé potentiel pour un agent pharmacologique utile dans le traitement du cancer du testicule, notamment d'une tumeur germinale du testicule.

6. Procédé pour évaluer chez un sujet l'effet d'un traitement pour le cancer du testicule, notamment pour une tumeur germinale du testicule, comprenant les étapes:
a) déterminer le niveau d'expression du gène encodant le peptide pro-EPIL dans un échantillon biologique isolé à partir dudit sujet ;
b) déterminer un second niveau d'expression du gène encodant le peptide pro-EPIL dans un échantillon biologique isolé à partir dudit sujet ;
c) comparer les premier et second taux du niveau d'expression du gène encodant le peptide pro-EPIL dans un échantillon biologique isolé à partir dudit sujet ;
**caractérisé en ce qu'**une diminution du niveau d'expression du gène encodant le peptide pro-EPIL dans le second échantillon biologique isolé à partir dudit sujet indique la régression dudit cancer du testicule, notamment d'une tumeur germinale du testicule.

7. Procédé selon l'une quelconque des revendications 1 à 6, **caractérisé en ce que** le niveau d'expression est déterminé en détectant la présence, l'absence ou le niveau d'ARNm transcrit à partir dudit gène ou du polypeptide encodé par ledit gène, ou d'un fragment spécifique de celui-ci.

8. Procédé selon la revendication 7, **caractérisé en ce que** ledit polypeptide est détecté ou quantifié par analyse par Western blot, par chromatographie, par dosage immunologique ou par immunohistochimie.

9. Procédé selon l'une quelconque des revendications 1 à 8, **caractérisé en ce que** l'échantillon biologique obtenu à partir du sujet est choisi dans le groupe comprenant le sang total, le sérum ou le plasma sanguin, la biopsie tumorale ou des combinaisons de ceux-ci.

10. Kit pour détecter et/ou classer un cancer du testicule, notamment une tumeur germinale du testicule, comprenant :
a) un anticorps spécifiquement dirigé contre le peptide pro-EPIL ;
b) un anticorps spécifiquement dirigé contre la HCG ou sa sous-unité polypeptidique HCGβ ; et
c) un anticorps spécifiquement dirigé contre l'AFP polypeptidique humaine.

11. Kit pour détecter et/ou classer un cancer du testicule, notamment une tumeur germinale du testicule, comprenant :
a) un ensemble d'amorces capables d'amplifier spécifiquement l'ARN ou l'ADNc du pro-EPIL ; et
b) un ensemble d'amorces capables d'amplifier spécifiquement l'ARN ou l'ADNc du groupe consistant en la HCG, sa sous-unité HCGβ ; et
c) un ensemble d'amorces capables d'amplifier l'ARN ou l'ADNc de l'alpha-foetoprotéine humaine AFP.

12. Kit pour détecter et/ou classer un cancer du testicule, notamment une tumeur germinale du testicule, consistant en :
a) une sonde nucléique capable de s'hybrider spécifiquement avec l'ARN du pro-EPIL ;
b) une sonde nucléique capable de s'hybrider spécifiquement avec l'ARN de la gonadotrophine humaine HCG ou de sa sous-unité bêta HCGβ ; et
c) une sonde nucléique capable de s'hybrider spécifiquement avec l'ARN de l'alpha-foetoprotéine humaine AFP.

13. Biopuce en phase solide pour détecter et/ou classer un cancer du testicule, notamment une tumeur germinale du testicule, consistant en :
a) une molécule d'acide nucléique capable de s'hybrider spécifiquement avec l'ARN du pro-EPIL ;
b) une molécule d'acide nucléique capable de s'hybrider spécifiquement avec l'ARN de la gonadotrophine humaine HCG ou de sa sous-unité bêta HCGβ ; et
c) une molécule d'acide nucléique capable de s'hybrider spécifiquement avec l'ARN de l'alpha-foetoprotéine humaine AFP.

14. Puce à protéines en phase solide pour détecter et/ou classer un cancer du testicule, notamment une tumeur germinale du testicule, comprenant :
a) un anticorps spécifiquement dirigé contre le peptide pro-EPIL ;
b) un anticorps spécifiquement dirigé contre la HCG ou sa sous-unité polypeptidique HCGβ ; et
c) un anticorps spécifiquement dirigé contre l'AFP polypeptidique humaine,
fixé à un substrat solide.

15. Utilisation de l'expression du gène pro-EPIL comme biomarqueur pour le diagnostic d'un cancer du testicule, notamment d'une tumeur germinale du testicule.


### SEQUENCE LISTING

<110> UNIVERSITE PARIS DESCARTES
   BELLET, DOMINIQUE
   PECKING, ALAIN
   RICHON, SOPHIE
   PETRAGLIA, FELICE
<120> Pro-EPIL expression level in a biological sample as testicular cancer biomarker, particularly in combination with the HCGbeta and AFP biomarkers
<130> 354601 D26505
<150> US 61/048,412
   <151> 2008-04-28
<160> 11
<170> PatentIn version 3.3
<210> 1
   <211> 139
   <212> PRT
   <213> Homo sapiens
<400> 1
   Met Ala Ser Leu Phe Arg Ser Tyr Leu Pro Ala Ile Trp Leu Leu Leu
   1 5 10 15
   Ser Gln Leu Leu Arg Glu Ser Leu Ala Ala Glu Leu Arg Gly Cys Gly
   20 25 30
   Pro Arg Phe Gly Lys His Leu Leu Ser Tyr Cys Pro Met Pro Glu Lys
   35 40 45
   Thr Phe Thr Thr Thr Pro Gly Gly Trp Leu Leu Glu Ser Gly Arg Pro
   50 55 60
   Lys Glu Met Val Ser Thr Ser Asn Asn Lys Asp Gly Gln Ala Leu Gly
   65 70 75 80
   Thr Thr Ser Glu Phe Ile Pro Asn Leu Ser Pro Glu Leu Lys Lys Pro
   85 90 95
   Leu Ser Glu Gly Gln Pro Ser Leu Lys Lys Ile Ile Leu Ser Arg Lys
   100 105 110
   Lys Arg Ser Gly Arg His Arg Phe Asp Pro Phe Cys Cys Glu Val Ile
   115 120 125
   Cys Asp Asp Gly Thr Ser Val Lys Leu Cys Thr
   130 135
<210> 2
   <211> 22
   <212> PRT
   <213> Homo sapiens
<400> 2
   Met Ala Ser Leu Phe Arg Ser Tyr Leu Pro Ala Ile Trp Leu Leu Leu
   1 5 10 15
   Ser Gln Leu Leu Arg Glu
   20
<210> 3
   <211> 30
   <212> PRT
   <213> Homo sapiens
<400> 3
   Ser Leu Ala Ala Glu Leu Arg Gly Cys Gly Pro Arg Phe Gly Lys His
   1 5 10 15
   Leu Leu Ser Tyr Cys Pro Met Pro Glu Lys Thr Phe Thr Thr
   20 25 30
<210> 4
   <211> 50
   <212> PRT
   <213> Homo sapiens
<400> 4
   Leu Glu Ser Gly Arg Pro Lys Glu Met Val Ser Thr Ser Asn Asn Lys
   1               5                   10                  15
   Asp Gly Gln Ala Leu Gly Thr Thr Ser Glu Phe Ile Pro Asn Leu Ser
               20                  25                  30
   Pro Glu Leu Lys Lys Pro Leu Ser Glu Gly Gln Pro Ser Leu Lys Lys
           35                  40                  45
   Ile Ile
       50
<210> 5
   <211> 25
   <212> PRT
   <213> Homo sapiens
<400> 5
   Ser Gly Arg His Arg Phe Asp Pro Phe Cys Cys Glu Val Ile Cys Asp
   1               5                   10                  15
   Asp Gly Thr Ser Val Lys Leu Cys Thr
               20                  25
<210> 6
   <211> 617
   <212> DNA
   <213> Homo sapiens
<400> 6
   agtctggagc ccagaaggga cacaccagca cagtctggta ggctacagca gcaagtctct    60
   aaagaaaggc tgagaacacc cagaacagga gagttcaggt ccaggatggc cagcctgttc   120
   cggtcctatc tgccagcaat ctggctgctg ctgagccaac tccttagaga aagcctagca   180
   gcagagctga ggggatgtgg tccccgattt ggaaaacact tgctgtcata ttgccccatg   240
   cctgagaaga cattcaccac caccccagga gggtggctgc tggaatctgg acgtcccaaa   300
   gaaatggtgt caacctccaa caacaaagat ggacaagcct taggtacgac atcagaattc   360
   attcctaatt tgtcaccaga gctgaagaaa ccactgtctg aagggcagcc atcattgaag   420
   aaaataatac tttcccgcaa aaagagaagt ggacgtcaca gatttgatcc attctgttgt   480
   gaagtaattt gtgacgatgg aacttcagtt aaattatgta catagtagag taatcatgga   540
   ctggacatct catccattct catatgtatt ctcaatgaca aattcactga tgcccaatta   600
   aatgattgct gtttaaa                                                  617
<210> 7
   <211> 417
   <212> DNA
   <213> Homo sapiens
<400> 7
   atggccagcc tgttccggtc ctatctgcca gcaatctggc tgctgctgag ccaactcctt    60
   agagaaagcc tagcagcaga gctgagggga tgtggtcccc gatttggaaa acacttgctg   120
   tcatattgcc ccatgcctga gaagacattc accaccaccc caggagggtg gctgctggaa   180
   tctggacgtc ccaaagaaat ggtgtcaacc tccaacaaca aagatggaca agccttaggt   240
   acgacatcag aattcattcc taatttgtca ccagagctga agaaaccact gtctgaaggg   300
   cagccatcat tgaagaaaat aatactttcc cgcaaaaaga gaagtggacg tcacagattt   360
   gatccattct gttgtgaagt aatttgtgac gatggaactt cagttaaatt atgtaca      417
<210> 8
   <211> 66
   <212> DNA
   <213> Homo sapiens
<400> 8
   atggccagcc tgttccggtc ctatctgcca gcaatctggc tgctgctgag ccaactcctt    60
   agagaa                                                               66
<210> 9
   <211> 90
   <212> DNA
   <213> Homo sapiens
<400> 9
   agcctagcag cagagctgag gggatgtggt ccccgatttg gaaaacactt gctgtcatat 60
   tgccccatgc ctgagaagac attcaccacc 90
<210> 10
   <211> 150
   <212> DNA
   <213> Homo sapiens
<400> 10
   ctggaatctg gacgtcccaa agaaatggtg tcaacctcca acaacaaaga tggacaagcc 60
   ttaggtacga catcagaatt cattcctaat ttgtcaccag agctgaagaa accactgtct 120
   gaagggcagc catcattgaa gaaaataata 150
<210> 11
   <211> 75
   <212> DNA
   <213> Homo sapiens
<400> 11
   agtggacgtc acagatttga tccattctgt tgtgaagtaa tttgtgacga tggaacttca 60
   gttaaattat gtaca 75